# EUROPEAN PATENT SPECIFICATION

(11) **EP 0 520 185 B1**
(45) Date of publication and mention of the grant of the patent: **28.02.1996**
(21) Application number: 92108434.9
(22) Date of filing: 19.05.1992
(51) Int. Cl.: G01N 33/49

(54) **Blood separation means**
Mittel zur Auftrennung von Blut
Moyen pour la séparation du sang

(30) Priority: 25.06.1991 JP 153236/91
(43) Date of publication of application: 30.12.1992
(73) Proprietor: NISSHO CORPORATION, Osaka-shi, Osaka-fu (JP)
(72) Inventor: Murakami, Kazunori, Kusatsu-shi, Shiga-ken (JP); Taguchi, Tetsuo, Yao-shi, Osaka-fu (JP)
(74) Representative: Kraus, Walter, Dr.

(56) References cited:
- EP-A- 0 075 119
- EP-A- 0 076 051
- EP-A- 0 384 331

## Description

### BACKGROUND OF THE INVENTION

### 1. Field of the Invention

The present invention relates to a blood component-separating composition ( hereinafter referred to as "blood separation means" ) which is employed in the centrifugal separation method to separate serum or plasma from whole blood, wherein the difference in specific gravity between them is utilized.

### 2. Prior Art

Many kinds of blood separation means which have been proposed are useful in the centrifugal method (cf. US-PS 5 304 605, US-PS 4 049 692, US-PS 4 083 784, US-PS 4 101 422, US-PS 4 148 764, US-PS 4 386 003, US-PS 4 457 782, US-PS 4 534 798, EP 0 035 575). Those prior art separation means contain as their main ingredient a gel-like material such as silicone oil, chlorinated polybutene, acrylic polymer or copolymer of α-olefin and diester of maleic acid. Typical additives blended with the main ingredient are: a thixotropic agent enhancing important properties to the gel-like material; and an inorganic substance. The thixotropic agent causes the gel-like material not to flow within blood collection tubes but stay on its bottom while being transported. When centrifugal force is applied to the collection tubes filled with blood, the gel-like material moves upwards and forms a partition barrier between the serum ( or plasma ) and the clot. The thixotropic agent also enhances a stable strength to the partition barrier. On the other hand, the inorganic substance such as titanium dioxide and calcium carbonate is blended with the gel-like material so as to adjust its specific gravity.

The molecules of the thixotropic agents in the prior art blood separation means have in general functional groups capable of forming hydrogen bonds. The thixotropic agents are thus either inorganic fine powders such as silica and clay, or organic gelling agents.

There are observed many disadvantages inherent in the prior art thixotropic agents, as summarized below. The inorganic powder such as silica has a specific density which is too high compared with that of the gel-like material used as the main ingredient. If the inorganic powder is blended at a content enough to realize a required level of viscosity, then the specific gravity of the blood separation means will rise often above a desirable range of 1.035 to 1.060. On the contrary, the specific gravity adjusted to fall within this range will be accompanied by an undesirably low viscosity. Thus, it has been difficult to meet the requirement on the specific gravity concurrently with the requirement of the viscosity. It is another problem that, due to the high specific gravity of inorganic powder, the actual specific gravity of the blood separation means cannot be kept at a constant level but varies among production lots thereof and in course of time. In addition, poor compatibility of a rich content of inorganic powder with the gel-like material will cause some fractions of the separation means to be dispersed in the serum phase ( or plasma phase ), forming an oily substance which floats therein. Such floating particles are likely to clog the nozzles in an automatic analyzer. Further, because of insufficient strength of the partition barrier, perfect centrifugal isolation of the serum ( or plasma ) from the clot is not ensured. A number of blood cells will remain in the serum phase above the partition, thus impairing the separation accuracy.

In a case wherein the thixotropic agent added in the blood separation means is an organic gelling agent, for example a sorbitol-aromatic aldehyde condensation product, a very low content suffices for a desired level of thixotropy. The extremely low content however brings about inevitably a large variation of viscosity between the production lots of said separation means. In use of such a blood separation means, it forms an unstable partition barrier. Moreover, the intermolecular attraction of such an organic gelling agent becomes intensive in course of time to such a degree as increasing the minimum shear stress for solation of the separation means. This means a poor stability during storage, and the blood separation means after storage for a long time will not smoothly rise ( or "ascend" ) within the centrifuged collection tube, thus lowering its separation capability.

EP-A-0 076 051 relates to a method of making a thixotropic gel for partitioning separated phases of a fluid sample, the phases having differing densities and the gel having a density intermediate those of the phases, wherein a silicone fluid, a filler material and a network former are reacted to form the gel, which is characterised in that the silicone fluid has a first viscosity and comprises a single stock fluid or a blend comprising at least two blend stock fluids, each said stock fluid in the blend having a viscosity within about ±15% of said first viscosity, and said gel-forming reaction is carried out at or below about 120°C. The gel produced is primarily intended for partitioning the serum and clot portions of a blood sample.

EP-A-0 075 119 discloses thixotropic agents for use in unsaturated polyester resins, which agent can be a combination of a cyclohexamide of a saturated fatty acid of at least 10 carbon atoms and preferably up to 30 carbon atoms and an oligomeric ester amide.

### SUMMARY OF THE INVENTION

Therefore, an object of the present invention is to provide a blood separation means free from the aforementioned drawbacks of the prior art separation means.

The blood separation means provided in the invention is characterized by a fatty acid amide(s) as the thixotropic agent which are added to a gel-like material and enhance thereto a thixotropic property.

### THE PREFERRED EMBODIMENTS

The inventors have conducted to achieve the object a series of researches and found the fact that the fatty acid amide(s) having a low specific gravity can be used as an effective thixotropic agent, which gives an advantage that the blood separation means can be easily and favorably controlled of not only its specific gravity but also of its viscosity at the same time. Unlike the prior art separation means employing the inorganic powder as thixotropic agent, any fractions of the present separation means do not form an oily substance floating within the phase of serum ( or plasma ). Owing to a sufficient strength of the partition barrier formed with the separation means, no blood cell remains within the serum. In contrast with the conventional organic gelling agent, the molecules of fatty acid amides do not tend to aggregate together during storage, thus never rendering it difficult for the separation means to rise in the centrifuged tubes. The blood separation

means of the invention well maintains its high separating capability for a long time, and is therefore of a higher practical value.

Now, the invention will be summarized below more in detail.

The thixotropic agent employed in this invention is any appropriate one of fatty acid amides inert to blood, or any mixture thereof. The number of carbon atoms per one molecule of the amides is from 10 to 25, and more desirably from 16 to 18.

With more than 25 atoms per molecule, the property of fatty acid amides will be affected adversely by an alkyl group included therein, thus making it difficult to render thixotropic the blood separation means. If however the molecule includes less than 10 atoms, then its melting point will be too low for the amides to be thermally stable, and the separation means could not be stored in a stable state for a long time.

The preferable content of fatty acid amides in the blood separation means is from 0.5 to 7 parts, or more desirably from 1 to 4 parts by weight for 100 parts by weight of a gel-like material as the main ingredient. In a case wherein less than 0.5 parts by weight of the amides are contained, the partition barrier will be weak and apt to be fluidized, thereby disabling the means to fully perform its separating function. Further, storage stability will also be insufficient because its state is not kept stable during storage. The amide content of more than 7 parts by weight will however render the separation means so flowable that it does not smoothly rise within the centrifuged tubes. Such an excessive quantity of the amides cannot be dispersed uniform in the gel-like material, also impairing the function of said separation means.

Any of usually used gel-like materials may be selected as the gel-like material in the invention insofar as they preferably have a specific gravity of 1.035 - 1.055 and viscosity of 30,000 - 150,000 mPa.s (cP), at 25 °C. With the specific gravity below 1.035 and the viscosity lower than 30,000 mPa.s (cP), the blood separation means is too mobile to stand still within the tubes when gravitational force or the like is imparted to it during storage. In detail, the separation means in this case may move close to a rubber stopper sealing an open top of the previously evacuated blood collection tube, even if received therein to rest on the bottom thereof at first. Consequently, it will not only be mixed with and thus contaminate a centrifugally separated serum or plasma, but also be left sticking to the rubber stopper, thus making it difficult to obtain a pure sample of serum or plasma. Such a separation means will rise away from the bottom at an accelerated timing so that the partition barrier is formed too early to prevent the blood cells from completely sinking below the barrier. Thus, the blood cells remaining within a fraction above the barrier will impair its separating capability.

On the contrary, a specific gravity above 1.055 in combination with a viscosity higher than 150,000 will disable the smooth rising of the separation means, also failing to enhance a desirable capability of separation. The excessively high viscosity will cause further disadvantages such as difficult handling and allocation of the separation means to a number of collection tubes.

A preferable example of the gel-like material is a copolymer ( or more exactly "terpolymer" in this case ) of sebacic acid with a mixture of 2,2-dimethyl-1,3-propanediol and 1,2-propanediol. Molar ratio of the mixture to sebacic acid forming the copolymer is desirably from 1.02 : 1 to 1.07 : 1.

The blood separation means in the invention may readily be produced for example by conducting the steps of: heating to a temperature between 60 °C and 80 °C a given amount of the gel-like material; adding thereto an appropriate amount of the fatty acid amide(s); and continuing to stir this mixture with a sufficient shearing stress, while keeping the temperature, until the amide(s) is completely dissolved in the gel-like material.

The blood separation means prepared in this manner in the invention need have a specific gravity intermediate those of the serum or plasma and the clot. The separation means put in blood collection tubes usually rests on the bottom thereof. Therefore, a larger difference in specific gravity between the separation means and the clot or blood cells is advantageous in that the centrifuged separation means can ascend within the tubes more smoothly and more rapidly. On the other hand, such a separation means as having an overall specific gravity not exceeding 1.035 includes its fraction whose specific gravity is much lower than 1.035. This fraction may undesirably migrate from said means into the centrifugally separated serum phase or plasma phase.

Besides the specific gravity as prescribed above, the blood separation means which is composed of the gel-like material and the predetermined amount of fatty acid amide(s) preferably has a viscosity from 100,000 to 400,000 mPa.s (cP) at 25 °C ( when measured in the same manner as will be described for the Examples given below ).

The specific gravity as well as viscosity of the separation means may be adjusted, if necessary, by further using the conventional thixotropic agent such as silica and/or the conventional gravity-adjusting inorganic substance such as titanium dioxide.

### Examples

The following Examples are given only by way of example, with no intention of delimiting thereto the scope of the present invention.

Viscosity of the Examples was measured using "E-Type Viscometer" which is a rotary viscometer ( with a cone angle at 3° and a diameter of 28 mm, made by TOKYO KEIKI CO., LTD. ). Specific gravity was measured according to the Cupric Sulfate Method using cupric sulfate solutions of different concentrations. One drop of each sample was put in the solutions, in order to find which of them neither caused the drop to rise to the surface nor sink to the bottom. The thus found solution ought to have the same specific gravity as the tested sample.

### -- Preparation of copolymers --

### Copolymer No. 1

Copolymer of sebacic acid may be produced by any conventional method known in this field of art.

Polymerisation was carried out in a quatre-mouthed flask comprising a stirrer, a thermometer, an N₂ gas-introducing tube and a Vigreaux column. This Vigreaux column of a medium length comprised in turn a distillation head and a condenser which was composed of a thermometer and a receptacle. The condenser was arranged to distill water and/or an amount of excessive diol, under atmospheric or reduced pressure. Reactants which were involved in this process are as follows.

202 grams of sebacic acid, 89 grams of 2,2-dimethyl-1,3-propanediol and 16 grams of 1,2-propanediol were put in the quatre-mouthed flask to thereby form a reaction mixture. This mixture was heated up to about 225 °C, by continuously removing water therefrom while maintaining the vapor temperature almost constant to fall within a range of about 100 - 120 °C. Upon detection of a reduced rate of water generation after about 4 hours from the start of reaction, a little amount of a titanium compound as esterification catalyst ( at a ratio corresponding to 0.005 % of initial weight of the reactants ) was added. At the same time, the reaction system pressure was reduced to 933-1333 kPa (70 - 100 mmHg), and reaction was continued for 5 hours under this condition. Subsequent to this phase of process, the pressure was reduced below 66.7 kPa (5 mmHg) for a further reaction for 3 hours. A highly viscous product was discharged from the flask and cooled to room temperature to give a yield of 98 %. This product's viscosity was 35,000 mPa.s (cP), and specific gravity was 1.041 at 25 °C.

### Copolymer No. 2

202 grams of sebacic acid, 87 grams of 2,2-dimethyl-1,3-propanediol and 15 grams of 1,2-propanediol were put in the quatre-mouthed flask to form another reaction mixture. Reaction to produce a copolymer was carried out in the same manner as for the copolymer No. 1, except that the total reaction time was 15 hours including the last phase for 3 hours with a pressure kept at 20 kPa (1.5 mmHg). The yield of copolymer No. 2 obtained in this manner was 95 %, its viscosity being 150,000 mPa.s (cP) with a specific gravity of 1.041 at 25 °C.

### Copolymer No. 3

202 grams of sebacic acid, 88 grams 2,2-dimethyl-1,3-propanediol and 16 grams of 1,2-propanediol were put in the quatre-mouthed flask to form still another reaction mixture. Reaction to produce a copolymer was carried out in the same manner as for the copolymer No. 1, except that the total reaction time was 15 hours including the last phase for 3 hours with a pressure kept at 20 kPa (1.5 mmHg). The yield of copolymer No. 3 obtained in this manner was 95 %, its viscosity being 68,000 mPa.s (cP) with a specific gravity of 1.041 at 25 °C.

### -- Preparation of blood separation means --

### Example No. 1

This example of blood separation means was prepared by blending 2 parts by weight of stearamide ( containing a small mixed amount of palmitamide ) with 100 parts by weight of the copolymer No. 1. Example No. 1 showed a viscosity of 140,000 mPa.s (cP) and specific gravity of 1.043 at 25 °C.

### Example No. 2

3 parts by weight of stearamide were blended with 100 parts by weight of the copolymer No. 1 to give Example No. 2 of blood separation means having a viscosity of 187,000 mPa.s (cP) and specific gravity of 1.042 at 25 °C.

### Example No. 3

2 parts by weight of stearamide were blended with 100 parts by weight of the copolymer No. 2 to give Example No. 3 of blood separation means having a viscosity of 260,000 mPa.s (cP) and specific gravity of 1.041 at 25 °C.

### Example No. 4

3 parts by weight of stearamide were blended with 100 parts by weight of the copolymer No. 3 to give Example No. 4 of blood separation means having a viscosity of 156,000 mPa.s (cP) and specific gravity of 1.042 at 25 °C.

### Example No. 5

4 parts by weight of stearamide were blended with 100 parts by weight of the copolymer No. 3 to give Example No. 4 of blood separation means having a viscosity of 187,000 mPa.s (cP) and specific gravity of 1.042 at 25 °C.

### Reference No. 1

0.01 parts by weight of stearamide were blended with 100 parts by weight of the copolymer No. 3 to give a reference of blood separation means having a viscosity of 80,000 mPa.s (cP) and specific gravity of 1.041 at 25 °C.

### Reference No. 2

10 parts by weight of stearamide were blended with 100 parts by weight of the copolymer No. 2 to give another reference having a viscosity of 460,000 mPa.s (cP) and specific gravity of 1.044 at 25 °C.

### Reference No. 3

2 parts by weight of fine silica ( "Aerosil 300", a trademark of Nippon Aerosil Co., Ltd. ) were blended with 100 parts by weight of the copolymer No. 1 to give still another reference having a viscosity of 100,000 mPa.s (cP) and specific gravity of 1.053 at 25 °C.

### -- Comparison of examples with references --

Examples Nos. 1 - 5 as well as References Nos. 1 - 3 were tested of their stability during storage and their capability of dividing blood phases.

### (1) Stability during storage

Blood collection/separation tubes made of glass and those made of polyethylene terephthalate, having an inner diameter of 13.6 mm, were used. A small amount of blood separation means of "Example" or "Reference" weighing 1.5 grams was put in each tube, and after kept at 25 °C for 24 hours, "flow distance" of said means was measured at different temperatures. The "flow distance" is a distance between an initial position of the blood separation means and a final position thereof which was measured after predetermined hours of storage had passed. A result of this test is given on Table 1.

As will be seen on the table, the blood separation means of the present invention was stabler even if stored for a long time, and less flowable when transported or handled otherwise, than the prior art blood separation means represented by the References.

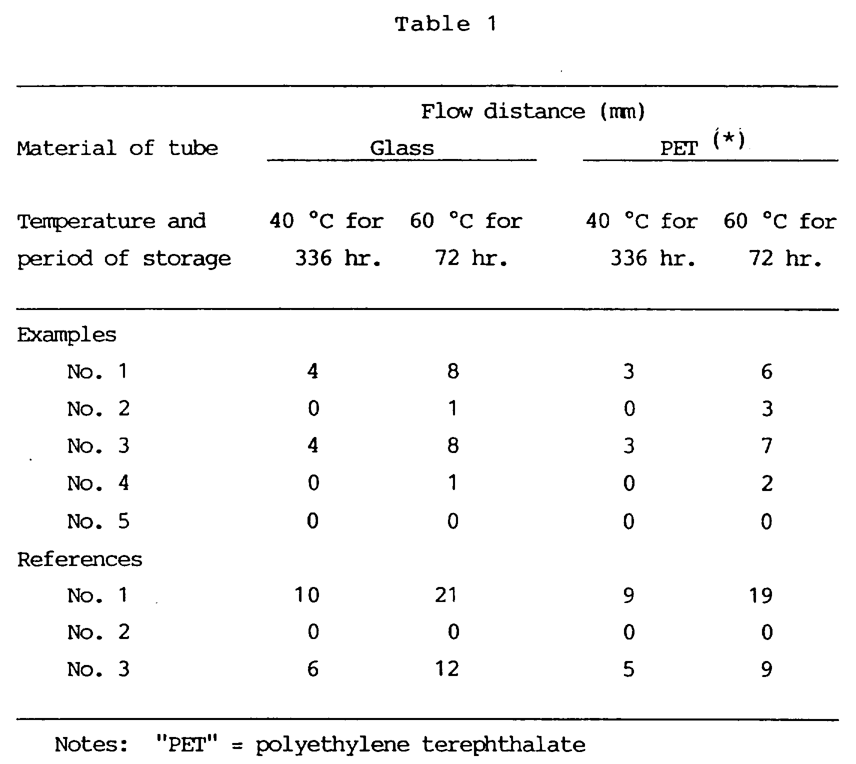

### (2) Capability of dividing blood phases

Similarly, blood collection/separation tubes made of glass and or polyethylene terephthalate, having an inner diameter of 13.6 mm, were used. A small amount of blood separation means of "Example" or "Reference" weighing 1.7 grams was put in each tube, and kept therein at 25 °C for 24 hours after storage at 40 °C for 336 hours.

9 ml of human whole blood was put in each tube, and after complete coagulation thereof, they were centrifuged with 1,300 G for 10 minutes ( "G" being the gravitational acceleration ).

Performance or capability of the blood separation means was evaluated as to the following items, according to the standards given below.

The term "ascendability" used herein indicates the extent to which the blood separation means can rise in the centrifuged collection tube previously filled with a given amount of human blood. A rating symbol "+++" ('excellent') was allotted to the separation means which completely rose within the blood collection tube, while another symbol "++" ('good') means a little amount of said means remaining on the tube bottom. A further rating symbol "+" ('poor') represents a significant amount of the blood separation means which was left on the bottom, whereas a still further symbol "±" ('worse') denotes the quite unsatisfactory rising of the separation means which fully remained on said tube bottom.

Further, the "stability of partition barrier" between the serum and the clot was judged based on the state of said barrier sticking to the tube wall, when 24 hours had passed after centrifugal separation process. Similarly to the "rising" property, the rating symbols "+++" (excellent), "++" (good), "+" (poor) and "±" (worse) respectively indicate: the perfectly sticking barrier; partially loosened barrier; significantly loosened barrier; and thoroughly loosened barrier.

The "released amount of oily substance" from the separation means was inspected by observation of the serum surface.

"Reddishness" of the serum was checked to determine whether or not any significant number of blood cells had been left in the serum, and also to determine whether or not hemolysis had occurred.

Test results are given on Tables 2 and 3 respectively for the glass tubes and for the polyethylene terephthalate tubes.

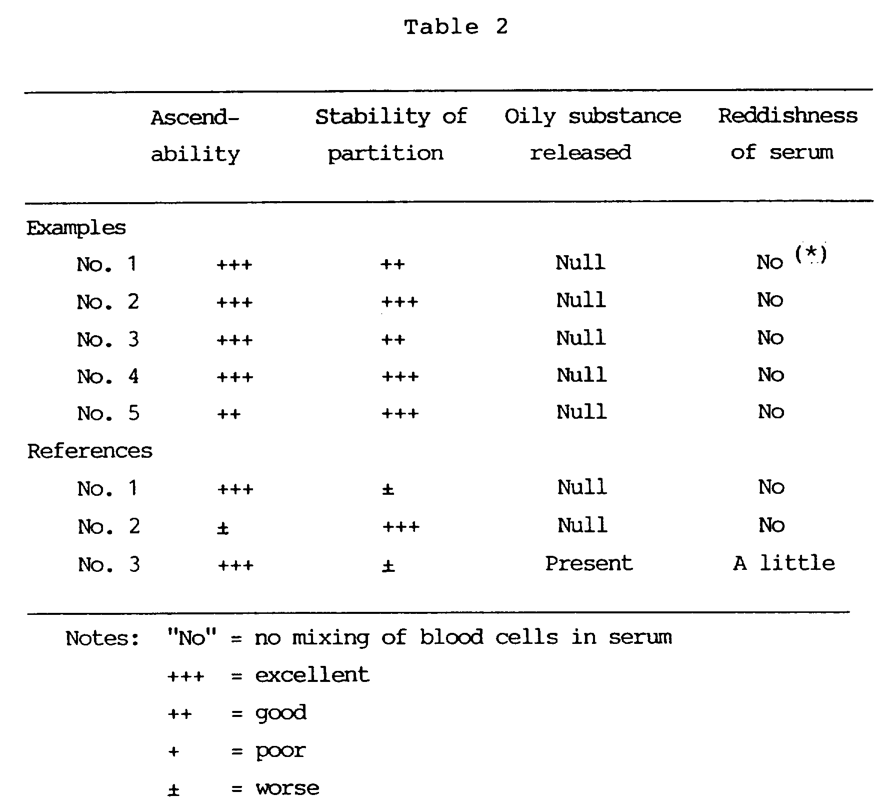

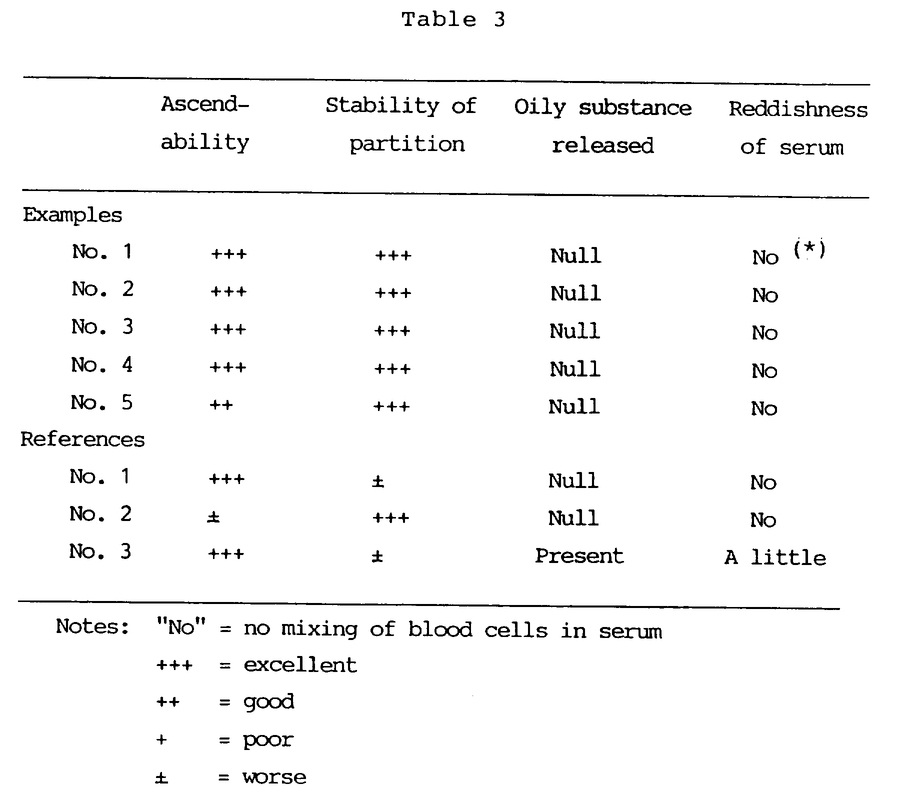

It will be seen on Tables 2 and 3 that in the blood phase-separating operation using the separation means provided in the invention, not only ascendability but also stability of partition barrier are excellent and satisfactory. Besides, there is neither observed any amount of oily substance released nor any extent of hemolysis or any number of blood cells remaining in the serum.

It will now be apparent from the foregoing that the blood separation means provided by the invention is advantageous in its high separating capability and its good stableness which are not affected by a long time reservation or the like. It does neither move adversely within the collection tubes during transportation thereof, nor change in its minimum shear stress and its rising property in the centrifugal process, thus maintaining its high capability of separating blood phases. Further, the separation means is inert to blood so that blood is neither absorbed nor hemolysis is caused by it. Radiation sterilization using gamma-ray or the like will not give rise to any physical or chemical change of the separation means. Further, the separation means will not release any oily substance which will give undesirable influence on the operation of testing apparatuses.

## Claims

1. A blood separation means comprising:
gel-like material as a main ingredient; and
an amount of a thixotropic agent added to and blended with the gel-like material,
wherein the thixotropic agent is a fatty acid amide or a mixture of fatty acid amides.

2. A blood separation means as defined in claim 1, wherein the fatty acid amides each contain 10 - 25 carbon atoms per molecule.

3. A blood separation means as defined in claim 2, wherein the fatty acid amides each contain 16 - 18 carbon atoms per molecule.

4. A blood separation means as defined in claim 1, 2 or 3, wherein 0.5 - 7 parts by weight of the fatty acid amides are blended with 100 parts by weight of the gel-like material.

5. A blood separation means as defined in claim 4, wherein 1 - 4 parts by weight of the fatty acid amides are blended with 100 parts by weight of the gel-like material.

## Patentansprüche

1. Mittel zur Trennung von Blut, enthaltend:
ein gelartiges Material als Hauptbestandteil; und
eine Menge an thixotropem Mittel, das zu dem gelartigen Material zugegeben und damit vermischt wurde,
wobei das thixotrope Mittel ein Fettsäureamid oder ein Gemisch aus Fettsäureamiden ist.

2. Mittel zur Trennung von Blut nach Anspruch 1, dadurch **gekennzeichnet**, daß die Fettsäureamide je 10 bis 25 Kohlenstoffatome pro Molekül enthalten.

3. Mittel zur Trennung von Blut nach Anspruch 2, dadurch **gekennzeichnet**, daß die Fettsäureamide je 16 bis 18 Kohlenstoffatome pro Molekül enthalten.

4. Mittel zur Trennung von Blut nach Anspruch 1, 2 oder 3, dadurch **gekennzeichnet**, daß 0,5 bis 7 Gew.-Teile Fettsäureamide mit 100 Gew.-Teilen gelartigem Material vermischt worden sind.

5. Mittel zur Trennung von Blut nach Anspruch 4, dadurch **gekennzeichnet**, daß 1 bis 4 Gew.-Teile Fettsäureamide mit 100 Gew.-Teilen gelartigem Material vermischt werden.

## Revendications

1. Un moyen pour la séparation du sang, comprenant :
- un matériau analogue à un gel, en tant qu'ingrédient principal; et
- une quantité d'un agent thixotrope qui est ajouté et mélangé au matériau analogue à un gel, dans lequel l'agent thixotrope est un amide d'acide gras, ou un mélange d'amides d'acides gras.

2. Un moyen pour la séparation du sang selon la revendication 1, dans lequel les amides d'acides gras comportent chacun 10 à 25 atomes de carbone par molécule.

3. Un moyen pour la séparation du sang selon la revendication 2, dans lequel les amides d'acides gras comportent chacun 16 à 18 atomes de carbone par molécule.

4. Un moyen pour la séparation du sang selon la revendication 1, la revendication 2, ou la revendication 3, dans lequel 0,5 à 7 parties en poids d'amides d'acides gras sont mélangées avec 100 parties en poids, du matériau analogue à un gel.

5. Un moyen pour la séparation du sang selon la revendication 4, dans lequel 1 à 4 parties en poids, d'amides d'acides gras sont mélangées avec 100 parties en poids, du matériau analogue à un gel.
